# DEMANDE DE BREVET EUROPEEN

(11) **EP 1 192 941 A1**
(43) Date de publication de la demande: **03.04.2002**
(21) Numéro de dépôt: 01402445.9
(22) Date de dépôt: 24.09.2001
(51) Int. Cl.: A61K 7/50

(54) **Composition de lavage des fibres kératiniques contenant des alkylamidoéthersulfates, des tensioactifs anioniques et des polymères cationiques**

(30) Priorité: 28.09.2000 FR 0012377
(71) Demandeur: L'OREAL, 75008 Paris (FR)
(72) Inventeur: Maubru, Mireille, 748400 Chatou (FR); Beauquey, Bernard, 92110 Clichy (FR); Decoster, Sandrine, 95210 Saint Gratien (FR)
(74) Mandataire: Casalonga, Axel

(57) **Abrégé**

La présente invention concerne une composition de lavage des matières kératiniques comprenant, dans un milieu cosmétiquement acceptable, au moins un alkyl(C₈₋₃₀)amidoéthersulfate, au moins un tensioactif anionique choisi parmi les alkyl(C₆-C₂₄)sulfates, les alkyl(C₆-C₂₄)éthersulfates, les alkyl(C₆-C₂₄)éthercarboxylates et les alkyl(C₆-C₂₄)polyglycosides anioniques, et au moins un polymère cationique dont la densité de charge cationique est supérieure ou égale à 1 milliéquivalent par gramme. L'invention concerne aussi un procédé de traitement cosmétique des matières kératiniques mettant en oeuvre la composition selon l'invention, ainsi qu'une utilisation de cette composition comme shampoing.

## Description

L'invention est relative à une composition de lavage des matières kératiniques comprenant, dans un milieu cosmétiquement acceptable, au moins un alkyl(C₈₋₃₀)amidoéthersulfate, au moins un tensioactif anionique et au moins un polymère cationique, à un procédé de traitement cosmétique des matières kératiniques, et à une utilisation en tant que shampoing.

Les associations connues de tensioactifs anioniques et de polymères cationiques conduisent à des propriétés cosmétiques qui ne sont pas totalement satisfaisantes, notamment sur cheveux séchés.

La demanderesse a trouvé de manière surprenante que l'utilisation d'alkyl(C₈₋₃₀)amidoéthersulfate en association avec certains tensioactifs anioniques et certains polymères cationiques, dans un milieu cosmétiquement acceptable, améliorait les propriétés cosmétiques des cheveux séchés, notamment le lissage au toucher, la malléabilité et la brillance. En outre, cette formulation possède un excellent niveau de tolérance oculaire qui permet de l'utiliser notamment pour des shampoings destinés aux enfants.

La présente invention a donc pour objet une composition de lavage des matières kératiniques comprenant, dans un milieu cosmétiquement acceptable, au moins un alkyl(C₈₋₃₀)amidoéthersulfate, au moins un tensioactif anionique et au moins un polymère cationique.

Par milieu cosmétiquement acceptable, on entend un milieu compatible avec toutes les matières kératiniques telles la peau, les cheveux, les ongles, les cils et sourcils, les lèvres et toute autre zone du corps et du visage, mais aussi d'odeur, d'aspect et de toucher agréables.

Un autre objet de l'invention consiste en un procédé de traitement cosmétique des matières kératiniques mettant en oeuvre la composition susvisée.

L'invention a encore pour objet une utilisation de la composition selon l'invention comme shampoing.

D'autres objets, caractéristiques, aspects et avantages de l'invention apparaîtront encore plus clairement à la lecture de la description et des divers exemples qui suivent.

Selon l'invention, la composition de lavage comprend, dans un milieu cosmétiquement acceptable :
- au moins un alkyl(C₈₋₃₀)amidoéthersulfate,
- au moins un tensioactif anionique choisi parmi les alkyl(C₆-C₂₄)-sulfates, les alkyl(C₆-C₂₄)éthersulfates, les alkyl(C₆-C₂₄)éthercarboxylates et les alkyl(C₆-C₂₄)-polyglycosides anioniques, et
- au moins un polymère cationique dont la densité de charge cationique est supérieure ou égale à 1 milliéquivalent par gramme (méq/g).

Les alkyl(C₈₋₃₀)amidoéthersulfates que l'on peut utiliser dans la composition selon l'invention se présentent sous forme de sels de métaux alcalins comme le sodium, de métaux alcalino-terreux comme le magnésium, d'ammonium ou d'aminoalcools.

Le groupe alkyle comportant de 8 à 30 atomes de carbone, de préférence de 10 à 20 atomes de carbone peut être linéaire ou ramifié, et choisi notamment parmi les groupes lauryle, myristyle et palmityle.

Le nombre de motifs oxyde d'alkylène, de préférence oxyde d'éthylène, des alkyl(C₈₋₃₀)amidoéthersulfates convenant dans l'invention est compris entre 1 et 100, et de préférence entre 2 et 20.

De préférence, on peut utiliser dans la composition selon l'invention, comme alkyl(C₈-C₂₀)amidoéthersulfate, le lauryl/myristylamidoéthersulfate de sodium à 3 moles d'oxyde d'éthylène, vendu sous la dénomination Laural® AMS par la société CECA.

Les tensioactifs anioniques convenant dans la présente invention sont choisis parmi les alkyl(C₆-C₂₄) sulfates, les alkyl(C₆-C₂₄)éthersulfates, les alkyl(C₆-C₂₄)éthercarboxylates et les alkyl(C₆-C₂₄)-polyglycosides anioniques bien connus dans la technique, et de préférence parmi les alkyl(C₆-C₂₄)éthersulfates.

Ils se présentent sous forme de sels de métaux alcalins, par exemple de sodium et de potassium, de métaux alcalino-terreux, par exemple de magnésium, d'ammonium, ou d'aminoalcool. Les tensioactifs anioniques à motifs éthers comportent un nombre de motifs oxyde d'alkylène, de préférence oxyde d'éthylène, compris entre 1 et 100, et de préférence entre 2 et 20.

À titre d'exemples d'alkylpolyglycosides anioniques, on peut notamment citer les alkylglycoside-citrates, les alkylglycosidetartrates, et comme exemple d'alkyl(C₆-C₂₄)éthersulfate, le lauryléthersulfate de sodium (C12/C14 à 70/30) à 2,2 moles d'oxyde d'éthylène.

La composition de lavage selon l'invention comprend un ou plusieurs polymères cationiques dont la densité de charge cationique est supérieure ou égale à 1 milliéquivalent par gramme, de préférence comprise entre 1 méq/g et 8 méq/g.

La densité de charge cationique peut être déterminée selon la méthode Kjeldahl.

Les polymères cationiques ayant une densité de charge cationique supérieure ou égale à 1 méq/g utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupes cationiques et/ou des groupes ionisables en groupes cationiques.

Les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les copolymères vinylpyrrolidone/(méth)acrylate de dialkylaminoalkyle quaternisés ou non, tels que les produits vendus sous la dénomination "Gafquat® " par la Société ISP, comme par exemple Gafquat® 734, 755 ou HS100, ou bien le produit dénommé "Copolymère 937". Ces polymères sont décrits en détail dans les brevets français 2 077 143 et 2 393 573.
(2) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alkylés ou s'ils comportent une ou plusieurs fonctions amines tertiaires, quaternisés. De tels polymères sont notamment décrits dans les brevets français 2 252 840 et 2 368 508 ;
(4) les dérivés de polyaminoamides résultant de la condensation de polyalkylènes-polyamines avec des acides polycarboxyliques suivie d'une alkylation par des agents bifonctionnels. On peut citer, par exemple, les polymères acide adipique/diakylaminohydroxyalkyldialkylène-triamine dans lesquels le groupe alkyle comporte de 1 à 4 atomes de carbone et désigne de préférence méthyle, éthyle, propyle. De tels polymères sont notamment décrits dans le brevet français 1 583 363.
   Parmi ces dérivés, on peut citer plus particulièrement les polymères acide adipique/diméthylaminohydroxypropyl-diéthylène-triamine vendus sous la dénomination "Cartaretine® F, F4 ou F8" par la société Sandoz.
(5) les polymères obtenus par réaction d'une polyalkylène-polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
   Des polymères de ce type sont en particulier commercialisés sous la dénomination "Hercosett® 57" par la société Hercules Inc., ou bien sous la dénomination de "PD 170" ou "Delsette® 101" par la société Hercules dans le cas du copolymère d'acide adipique/époxypropyl-diéthylène-triamine.
(6) les cyclopolymères de méthyldiallylamine ou de diméthyldiallylammonium tels que les homopolymères ou copolymères comportant des motifs répondant aux formules (VI) ou (VI') : 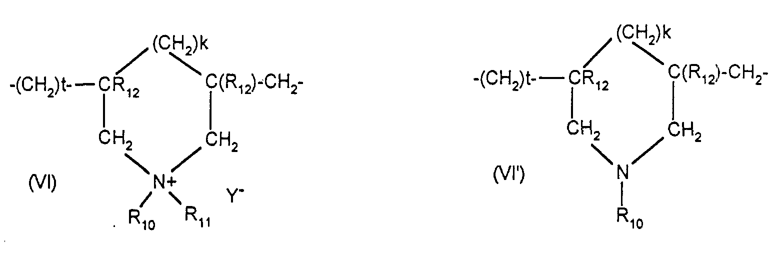 formules dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur, ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.
   On peut citer, par exemple, l'homopolymère de chlorure de diallyldiméthylammonium commercialisé sous la dénomination "MERQUAT® 100" par la société CALGON et les copolymères de chlorure de diallyldiméthylammonium et d'acrylamide commercialisés sous la dénomination "MERQUAT® 550".
(7) le polymère de diammonium quaternaire contenant des motifs récurrents répondant à la formule : 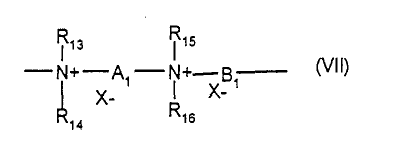 formule (VII) dans laquelle :
   R₁₃, R₁₄, R₁₅ et R₁₆, identiques ou différents, représentent des groupes aliphatiques, alicycliques, ou aryle aliphatiques contenant de 1 à 20 atomes de carbone ou des groupes hydroxyalkyle aliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-D ou -CO-NH-R₁₇-D où R₁₇ est un groupe alkylène et D un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique ;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement -(CH₂)ₙ-CO-D-OC-(CH₂)ₙ- dans lequel D désigne :
      a) un reste de glycol de formule : -O-Z-O-, où Z désigne un groupe hydrocarboné linéaire ou ramifié ou un groupement répondant à l'une des formules suivantes :

         -(CH₂-CH₂-O)x-CH₂-CH₂-

         -[CH₂-CH(CH3)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un groupe hydrocarboné linéaire ou ramifié, ou bien le groupe bivalent

         -CH₂-CH₂-S-S-CH₂-CH₂- ;
      d) un groupement uréylène de formule : -NH-CO-NH- ;

      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français numéros 2 320 330, 2 270 846, 2 316 271, 2 336 434 et 2 413 907 et les brevets US numéros 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4.026.945 et 4 027 020.
      On peut utiliser plus particulièrement les polymères qui sont constitués de motifs récurrents répondant à la formule : 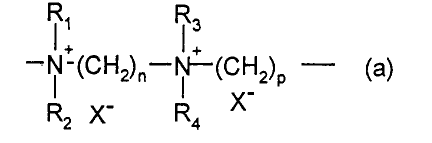 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
      Un composé de formule (a) particulièrement préféré est celui pour lequel R₁, R₂, R₃ et R₄ représentent un groupe méthyle et n = 3, p= 6 et X = Cl, dénommé "Hexadimethrine chloride" selon la nomenclature INCI (CTFA).
(8) les polymères de polyammonium quaternaires constitués de motifs de formule (VIII): 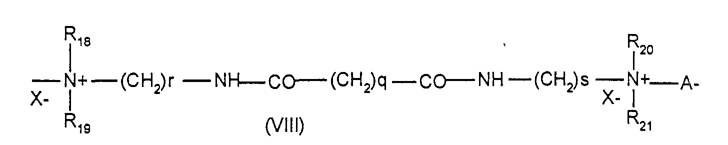 formule dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH,
      où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un groupe d'un dihalogénure ou représente de préférence -CH₂-CH₂-O-CH₂-CH₂-.

   De tels composés sont notamment décrits dans la demande de brevet EP-A-122 324.
   On peut, par exemple, citer parmi ceux-ci, les produits "Mirapol® A 15", "Mirapol® AD1", "Mirapol® AZ1" et "Mirapol® 175" vendus par la société Miranol.
(9) les homopolymères ou copolymères dérivés des acides acrylique ou méthacrylique et comportant des motifs : 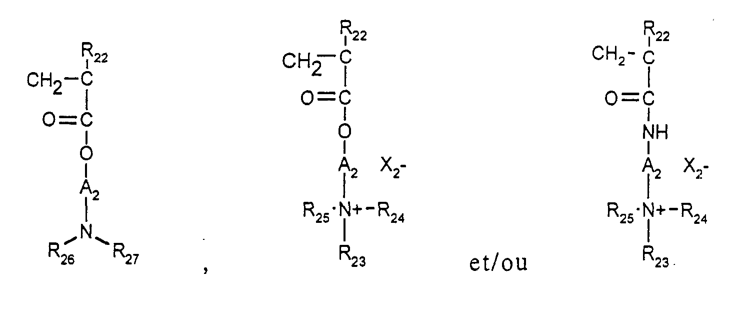 dans lesquels les groupements R₂₂ désignent indépendamment H ou CH₃,
   les groupements A₂ désignent indépendamment un groupe alkyle linéaire ou ramifié de 1 à 6 atomes de carbone, ou un groupe hydroxyalkyle de 1 à 4 atomes de carbone,
   les groupements R₂₃, R₂₄, R₂₅, identiques ou différents, désignant indépendamment un groupe alkyle de 1 à 18 atomes de carbone, ou un groupe benzyle,
   les groupements R₂₆ et R₂₇ représentent un atome d'hydrogène ou un groupement alkyle de 1 à 6 atomes de carbone,
   X₂⁻ désigne un anion, par exemple méthosulfate ou halogénure tel que chlorure ou bromure.
   Le ou les comonomères utilisables dans la préparation des copolymères correspondants appartiennent à la famille des acrylamides, méthacrylàmides, diacétone-acrylamides, acrylamides et méthacrylamides substitués à l'azote par des alkyle inférieurs, des esters d'alkyle, des acides acrylique ou méthacrylique, la vinylpyrrolidone ou des esters vinyliques.
(10) Les polymères quaternaires de vinylpyrrolidone et de vinylimidazole tels que, par exemple, les produits commercialisés sous les dénominations Luviquat® FC 905, FC 550 et FC 370 par la société B.A.S.F.
(11) Les polyamines comme le Polyquart® H vendu par HENKEL, référencé sous le nom de « POLYETHYLENEGLYCOL (15) TALLOW POLYAMINE » dans le dictionnaire CTFA.
(12) Les polymères réticulés de sels de méthacryloyloxyalkyl(C₁-C₄) trialkyl(C₁-C₄)ammonium tels que les polymères obtenus par homopolymérisation du méthacrylate de diméthylaminoéthyle quaternisé par le chlorure de méthyle, ou par copolymérisation de l'acrylamide avec le méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bis-acrylamide. On peut plus particulièrement utiliser un copolymère réticulé acrylamide/chlorure de méthacryloyloxyéthyl-triméthylammonium (20/80 en poids) sous forme de dispersion contenant 50 % en poids dudit copolymère dans de l'huile minérale. Cette dispersion est commercialisée sous le nom de "SALCARE® SC 92" par la Société ALLIED COLLOIDS. On peut également utiliser un homopolymère réticulé du chlorure de méthacryloyloxyéthyl-triméthylammonium contenant environ 50 % en poids de l'homopolymère dans de l'huile minérale ou dans un ester liquide. Ces dispersions sont commercialisées sous les noms de "SALCARE® SC 95" par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères, en particulier les polymères de chlorure de diméthyldiallylammonium ou les copolymères de chlorure de diméthyldiallylammonium et d'acrylamide, vendus sous les dénominations « MERQUAT® 100 » et « MERQUAT® 550 » par la société CALGON.

Comme autres polymères cationiques convenant dans la présente invention, on peut notamment citer les polymères cellulosiques, par exemple, les dérivés d'éthers de cellulose comportant des groupements ammonium quaternaires décrits dans le brevet français N° 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.

On peut également citer les copolymères de cellulose ou les dérivés de cellulose greffés avec un monomère hydrosoluble d'ammonium quaternaire, et décrits notamment dans le brevet US numéro 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium, de diméthyldiallylammonium.

Les produits commercialisés répondant à cette définition sont plus particulièrement les produits vendus sous la dénomination "Celquat® L 200" et "Celquat® H 100" par la Société National Starch.

La composition de lavage selon l'invention comprend les alkyl(C₈₋₃₀)amidoéthersulfates et tensioactifs anioniques tels que cités ci-dessus, en une quantité totale comprise entre 3 et 50 % en poids, et les polymères cationiques en une quantité comprise entre 0,001 et 10 % en poids, de préférence entre 0,05 et 5 % en poids, par rapport au poids total de la composition.

Le rapport en poids entre les tensioactifs anioniques et les alkyl(C₈₋₃₀)amidoéthersulfates est compris de préférence entre 100 et 0,01, de préférence entre 20 et 1.

Le milieu cosmétiquement acceptable peut être constitué par de l'eau ou par un mélange d'eau et d'un solvant cosmétiquement acceptable tel qu'un alcool inférieur en C₁-C₄, par exemple l'éthanol, l'isopropanol, le tertio-butanol, le n-butanol ; les alkylèneglycols comme le propylèneglycol, les éthers de glycol ; les alcanes en C₅-C₁₀ ; l'acétone, la méthyléthylcétone ; les acétates d'alkyle en C₁-C₄ comme l'acétate de méthyle, l'acétate d'éthyle, l'acétate de butyle ; le diméthoxyéthane, le diéthoxyéthane ; et leurs mélanges.

Le pH des compositions de l'invention est compris entre 4 et 8, de préférence entre 5 et 7.

Les compositions selon l'invention peuvent également contenir des additifs classiques bien connus dans la technique, tels que des épaississants polymériques naturels ou synthétiques, anioniques, amphotères, zwittérioniques, non-ioniques ou cationiques, associatifs
ou non, des épaississants non polymériques comme des acides ou des électrolytes, des nacrants, des opacifiants, des parfums, des huiles minérales, végétales et/ou synthétiques, des esters d'acides gras ou de polyéthylèneglycols, des colorants, des particules organiques ou minérales, des silicones volatiles ou non, modifiées ou non, des conservateurs, des agents de stabilisation du pH.

L'homme de métier veillera à choisir les éventuels additifs et leur quantité de manière à ce qu'ils ne nuisent pas aux propriétés des compositions de 1a présente invention.

Ces additifs sont présents dans la composition selon l'invention en une quantité allant de 0 à 20 % en poids par rapport au poids total de la composition.

Les compositions de lavage selon l'invention peuvent se présenter sous forme de liquides fluides ou épaissis, de gels, de crèmes, de mousses, d'émulsions simples ou d'émulsions multiples.

Elles peuvent être utilisées, par exemple, comme shampoings, soins rincés, masques de soin profond, gels douches, lotions ou crèmes de traitement du cuir chevelu.

La présente invention concerne également un procédé de traitement cosmétique des matières kératiniques qui consiste à appliquer une quantité efficace d'une composition de lavage telle que décrite ci-dessus, sur les matières kératiniques, à effectuer un rinçage après un éventuel temps de pose.

Selon un mode de réalisation préféré de l'invention, la composition peut être utilisée comme shampoing.

L'exemple suivant illustre la présente invention et ne doit être considéré en aucune manière comme limitant l'invention.

### Exemple

On a préparé un shampoing selon l'invention à partir des ingrédients suivants :

| | |
|---|---|
| Lauryléthersulfate de sodium (C12/C14 à 70/30) à 2,2 moles d'oxyde d'éthylène | 7,0 g |
| Lauryl/myristylamidoéthersulfate de sodium (3 moles d'oxyde d'éthylène) en solution aqueuse à 30 %, vendu par la société CECA sous la dénomination Laural® AMS | 11,4 g |
| Mélange de glycérides de palme oxyéthyléné (200 moles d'oxyde d'éthylène) et de coprah oxyéthyléné (7 moles d'oxyde d'éthylène) en suspension aqueuse à 70 %, vendu par la société GOLDSCHMIDT sous la dénomination Antil® 80 | 4,0 g |
| Mono/diglycérides de coprah oxyéthyléné (30 moles d'oxyde d'éthylène), vendu par la société GOLDSCHMIDT sous la dénomination Rewoderm® LI 63 | 4,0 g |
| Hydroxyéthylcellulose réticulée par l'épichlorhydrine et quaternisée par la triméthylamine, vendue par la société Union Carbide sous la dénomination JR 400 | 0,25 g |
| Dioléate de polyéthylèneglycol (55 moles d'oxyde d'éthylène) et de propylèneglycol en solution hydroalcoolique, vendu par la société GOLDSCHMIDT sous la dénomination Antil® 141 Liquid | 2,0 g |
| Acide citrique qsp | pH 7 |
| Conservateurs | qs |
| Eau déminéralisée qsp | 100,0 g |

On applique le shampoing selon l'invention sur les cheveux, on rince et on fait sécher les cheveux.

On observe un toucher lisse, une grande malléabilité et une brillance des cheveux. Cette composition présente une bonne tolérance oculaire.

## Revendications

1. Composition de lavage des matières kératiniques, **caractérisée en ce qu'**elle comprend, dans un milieu cosmétiquement acceptable,
- au moins un alkyl(C₈₋₃₀)amidoéthersulfate,
- au moins un tensioactif anionique choisi parmi les alkyl(C₆-C₂₄)-sulfates, les alkyl(C₆-C₂₄)éthersulfates, les alkyl(C₆-C₂₄)éther-carboxylates et les alkyl(C₆-C₂₄)polyglycosides anioniques, et
- au moins un polymère cationique dont la densité de charge cationique est supérieure ou égale à 1 milliéquivalent par gramme.

2. Composition selon la revendication 1, **caractérisée en ce que** les alkyl(C₈₋₃₀)amidoéthersulfates et lesdits tensioactifs anioniques se présentent sous la forme de sels de métaux alcalins ou alcalino-terreux, d'ammonium ou d'aminoalcools.

3. Composition selon la revendication 1 ou 2, **caractérisée en ce que** l'alkyl(C₈₋₃₀)amidoéthersulfate est le lauryl/myristylamidoéther-sulfate de sodium à 3 moles d'oxyde d'éthylène.

4. Composition selon l'une quelconque des revendications précédentes, **caractérisée en ce que** les tensioactifs anioniques sont choisis parmi les alkyl(C₆-C₂₄)éthersulfates.

5. Composition selon l'une quelconque des revendications précédentes, **caractérisée en ce que** les alkyl(C₈₋₃₀)amidoéthersulfates et lesdits tensioactifs anioniques sont présents en une quantité totale comprise entre 3 et 50 % en poids, par rapport au poids total de la composition.

6. Composition selon l'une quelconque des revendications précédentes, **caractérisée en ce que** le rapport en poids entre lesdits tensioactifs anioniques et les alkyl(C₈₋₃₀)amidoéther-sulfates est compris entre 100 et 0,01.

7. Composition selon la revendication 6, **caractérisée en ce que** le rapport entre lesdits tensioactifs anioniques et les alkyl(C_{8 30})amidoéthersulfates est compris entre 20 et 1.

8. Composition selon l'une quelconque des revendications précédentes, **caractérisée en ce que** le polymère cationique présentant une densité de charge cationique supérieure ou égale à 1 milliéquivalent par gramme, est choisi parmi :
- les copolymères vinylpyrrolidone/(méth)acrylate de dialkylamino-alkyle quaternisés ou non ;
- les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères ;
- les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alkylés ou s'ils comportent une ou plusieurs fonctions amines tertiaires, quaternisés ;
- les dérivés de polyaminoamides résultant de la condensation de polyalkylènes-polyamines avec des acides polycarboxyliques suivie d'une alkylation par des agents bifonctionnels.
- les polymères obtenus par réaction d'une polyalkylène-polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone ; le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1 ;
- les homopolymères ou copolymères comportant des motifs répondant aux formules (VI) ou (VI') : 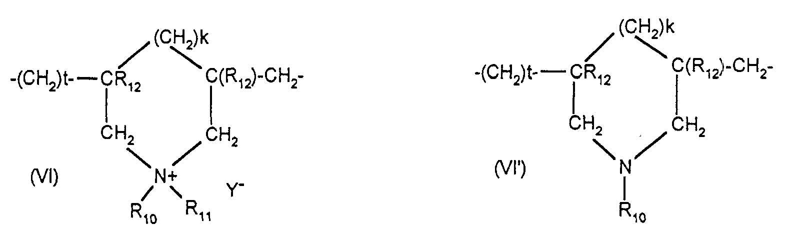 formules dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur, ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate ;
- le polymère de diammonium quaternaire contenant des motifs récurrents répondant à la formule : 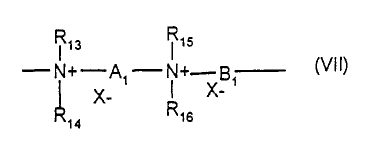 formule (VII) dans laquelle :
R₁₃, R₁₄, R₁₅ et R₁₆, identiques ou différents, représentent des groupes aliphatiques, alicycliques, ou aryle aliphatiques contenant de 1 à 20 atomes de carbone ou des groupes hydroxyalkyle aliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-D ou -CO-NH-R₁₇-D où R₁₇ est un groupe alkylène et D un groupement ammonium quaternaire ;
A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique ;
A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement -(CH₂)ₙ-CO-D-OC-(CH₂)ₙ- dans lequel D désigne :
a) un reste de glycol de formule : -O-Z-O-, où Z désigne un groupe hydrocarboné linéaire ou ramifié ou un groupement répondant à l'une des formules suivantes :
-(CH₂-CH₂-O)x-CH₂-CH₂-
-[CH₂-CH(CH3)-O]_{y}-CH₂-CH(CH₃)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-,
où Y désigne un groupe hydrocarboné linéaire ou ramifié, ou bien le groupe bivalent
-CH₂-CH₂-S-S-CH₂-CH₂-;
d) un groupement uréylène de formule : -NH-CO-NH- ;
- les polymères de polyammonium quaternaires constitués de motifs de formule (VIII): 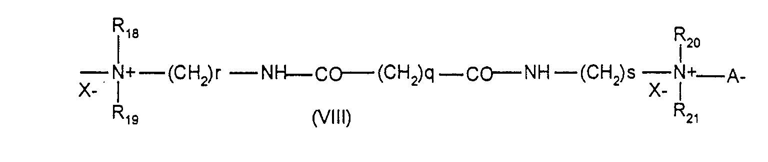 formule dans laquelle :
R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH,
où p est égal à 0 ou à un nombre entier compris entre 1 et 6, sous réserve que R₁₈, R₁₉, R₂₀ et R₂₁ ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un groupe d'un dihalogénure ou représente de préférence -CH₂-CH₂-O-CH₂-CH₂-.
- les homopolymères ou copolymères dérivés des acides acrylique ou méthacrylique et comportant des motifs : 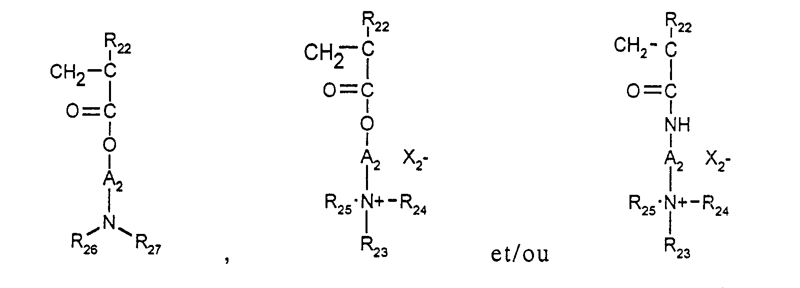 dans lesquels les groupements R₂₂ désignent indépendamment H ou CH3,
les groupements A₂ désignent indépendamment un groupe alkyle linéaire ou ramifié de 1 à 6 atomes de carbone, ou un groupe hydroxyalkyle de 1 à 4 atomes de carbone,
les groupements R₂₃, R₂₄, R₂₅, identiques ou différents, désignant indépendamment un groupe alkyle de 1 à 18 atomes de carbone, ou un groupe benzyle,
les groupements R₂₆ et R₂₇ représentent un atome d'hydrogène ou un groupement alkyle de 1 à 6 atomes de carbone,
X₂⁻ désigne un anion, par exemple méthosulfate ou halogénure tel que chlorure ou bromure ;
- les polymères quaternaires de vinylpyrrolidone et de vinylimidazole;
- les polymères réticulés de sels de méthacryloyloxyalkyl(C₁-C₄) trialkyl(C₁-C₄)ammonium ;
- les polyalkylèneimines, les polymères contenant des motifs vinylpyridine ou vinylpyridinium, des condensats de polyamines et d'épichlorhydrine, des polyuréylènes quaternaires et les dérivés de la chitine.

9. Composition selon l'une quelconque des revendications 1 à 7, **caractérisée en ce que** le polymère cationique présentant une densité de charge cationique supérieure ou égale à 1 milliéquivalent par gramme, est choisi parmi les dérivés d'éther de cellulose comportant des groupements ammonium quaternaires, les copolymères de cellulose
ou les dérivés de cellulose greffés avec un monomère hydrosoluble d'ammonium quaternaire, et leurs mélanges.

10. Composition selon l'une quelconque des revendications précédentes, **caractérisée en ce que** le polymère cationique est présent en une quantité comprise entre 0,001 et 10 % en poids, par rapport au poids total de la composition.

11. Composition selon la revendication 10, **caractérisée en ce que** le polymère cationique est présent en une quantité comprise entre 0,05 et 5 % en poids, par rapport au poids total de la composition.

12. Composition selon l'une quelconque des revendications précédentes, **caractérisée en ce que** le milieu cosmétiquement acceptable est constitué par de l'eau ou par un mélange d'eau et d'un solvant cosmétiquement acceptable.

13. Composition selon l'une quelconque des revendications précédentes, **caractérisée en ce qu'**elle comprend des additifs tels que des épaississants polymériques naturels ou synthétiques, anioniques, amphotères, zwittérioniques, non-ioniques ou cationiques, associatifs
ou non, des épaississants non polymériques comme des acides ou des électrolytes, des nacrants, des opacifiants, des parfums, des huiles minérales, végétales et/ou synthétiques, des esters d'acides gras ou de polyéthylèneglycols, des colorants, des particules organiques ou minérales, des silicones volatiles ou non, modifiées ou non, des conservateurs, des agents de stabilisation du pH.

14. Procédé de traitement cosmétique des matières kératiniques, **caractérisé en ce que** l'on applique sur les matières kératiniques une composition de lavage selon l'une quelconque des revendications précédentes.

15. Utilisation d'une composition de lavage selon l'une quelconque des revendications 1 à 13, comme shampoing.
